# EUROPÄISCHE PATENTSCHRIFT

(11) **EP 0 754 473 B1**
(45) Veröffentlichungstag und Bekanntmachung des Hinweises auf die Patenterteilung: **10.09.2003**
(21) Anmeldenummer: 96110700.0
(22) Anmeldetag: 03.07.1996
(51) Int. Cl.: A61N 5/10

(54) **Flexible, anpassbare Kunststoff-Körper mit Einzelkathetern oder äquidistant eingebetteten Kathetern oder Hülsen zur Einführung von Kathetern für die Strahlentherapie**
Plastic adaptable, flexible material comprising an only catheter or equidistant embedded catheters or envelopes to guide catheters in the field of radiotherapy
Des corps en plastique adaptables et flexibles comportant un cathéter ou des cathéters scellés de façon équidistante ou des enveloppes destinées à l'introduction des cathéters dans le domaine de la radiothérapie

(30) Priorität: 21.07.1995 DE 19526680
(43) Veröffentlichungstag der Anmeldung: 22.01.1997
(73) Patentinhaber: KABE Labortechnik GmbH, 51588 Nümbrecht-Elsenroth (DE)
(72) Erfinder: Kronholz, Hans, Priv.-Doz. Dr., 48249 Dülmen (DE); Schmilowski, Michael, Dr., 48159 Münster (DE); Anders, Christine, Dr., 45721 Haltern (DE); Brathun, Reinhold, 45721 Haltern (DE); Heinrich, Lothar, Dr., 48165 Münster (DE); Willich, Normann, Prof. Dr., 48167 Münster (DE)
(74) Vertreter: Christophersen & Partner

(56) Entgegenhaltungen:
- WO-A-93/14483
- US-A- 3 745 590
- US-A- 4 622 034
- US-A- 4 706 652
- US-A- 4 963 128

## Beschreibung

Für die Behandlung von Tumoren hat sich die Strahlentherapie bewährt. Für die Strahlenbehandlung in Körperhöhlen wie z. B. im Rachen, Darm oder Vaginalbereich sowie in operativ eröffneten Körperbereichen ist die Brachytherapie zu einer anerkannten Methode geworden, bei der die Strahlenquelle (-quellen), durch ein Afterloading-Gerät gesteuert, präzise und dosiert an den Behandlungsort im Körper gebracht und so bewegt werden, daß sich ein vorausberechneter Isodosenverlauf ergibt, so beschrieben in D. E. Wazer, R. Schmidt-Ullrich, W. Chasin, A. Wu, M. Buscher, Am. J. Otolaryngology 10 (3), (1989), 173 und R. Stepan, P. Lukas, U. Fink, P. Kneschaurek, Ir. Siewert, M. Molis, «Intraoperative Radiotherapy with High Dose Afterloading (Flabs Method) in Colorectal Carcinoma», in F.W. Schildberg, N. Willich, H.-J. Krämling (Herausgeber) «Intraoperative Radiation Therapy», Proceedings 4th International Symposium IORT, Munich 1992, Verlag Die Blaue Eule, Essen.

Um Schäden bei den Patienten zu vermeiden und die Voraussetzungen für eine exakte Bestrahlung sicherzustellen, müssen die Katheter genau positioniert und am oder im Körper auch fixiert werden. Nur wenn dies sichergestellt ist, kann eine Programmierung des gewünschten Isodosenverlaufs erfolgen und eine vorgeplante Bestrahlung auch gewährleistet werden.

Darüber hinaus muß sichergestellt sein, daß die Beweglichkeit der durch die Katheter geführten Strahlungsquellen weder durch Knicke, Einschnürungen noch durch zu starke Krümmungsradien der Katheter beeinträchtigt wird. Im Falle eines Festsitzens der Bestrahlungsquelle kann es zu erheblichen Überdosierungen mit der Gefahr der Schädigung des Patienten kommen. Bei den dann erforderlichen Notmaßnahmen ist eine Strahlenexposition des Personals unvermeidlich (Isabel Gosh, Sicherheitstechnisch bedeutsame Ereignisse an Afterloadinganlagen: Untersuchungen zur Strahlenexposition, Folgerungen zur Sicherheit von Personal und Patient, Diplomarbeit Berufsakademie Karlsruhe 1991).

Im Falle wiederholter Strahlenbehandlungen, jeweils mit abgeschwächter Strahlungsdosis, kommt der sicheren Fixierung der Katheter am Behandlungsort über einen größeren Zeitraum besondere Bedeutung zu. Auch um Patienten, die in größerer Entfernung zu den strahlungstherapeutischen Zentren einliegen, auf diese Behandlung extern und disponibel vorbereiten zu können, bedarf es einer exakten Katheterfixierung. Dadurch kann andererseits ein weitaus größerer Patientenkreis als bisher der strahlentherapeutischen Behandlung zugeführt werden.

Übliche derzeitige Praxis zur Herstellung von fixierten Katheter-Sätzen ist, Gummi-ähnliche Standard-Flachblöcke (1 bis 3 cm hoch, Fläche: 30 x 30 cm², Bezugsquellen sind z. B. Quandt Medizintechnik, Hamburg, oder Mick Radio-Nuclear Instruments Inc., Bronx, New York) zurechtzuschneiden und anschließend von Hand mit einer Hohlnadel zu durchbohren, um sie dann mit den Kathetern auszurüsten. Diese Arbeiten sind zeitaufwendig, binden Personal und führen nicht zu einer exakten Katheter-Einbettung (mit zunehmender Nadellänge nimmt die Reibung an der Nadelaußenwand zu, das Material des Flachblocks verformt sich, die Parallelität der Stichkanäle geht verloren). Darüber hinaus lassen sich diese sog. Flabs nicht sicher am vorgesehenen Applikationsort fixieren; es kommt zu Einrissen in den Durchstichen der Fixierfäden und so zur Verschiebung bis gänzlichen Ablösung des Flabs.
Verändern sich die Katheter aber in ihrer Lage insgesamt oder in der Lage zueinander, ist die Bestrahlung hinfällig. Es muß eine neue Lokalisation zur Eruierung der genauen Katheterverlagerung durchgeführt werden, oft ist dann eine Reoperation mit allen damit verbundenen zusätzlichen Risiken erforderlich. Bei gesicherter Katheterfixierung dagegen könnte eine Betreuung eines Teils der Patienten zwischen den Bestrahlungen auch im Heimatkrankenhaus erfolgen (Kostensenkung).
Zylindrische Kunststoff-Katheter-Systeme stehen ebenfalls nicht zur Verfügung. Man behilft sich mit selbst durchbohrten Kunststoff-Teilen, so beschrieben in oben zitierter Publikation von D. E. Wazer et al, S. 177. Im Rachenraum behilft man sich mit der Fixierung einzelner Katheter, die mit Ankern ausgerüstet sind und zeitaufwendig sowie für den Patienten erheblich belastend vernäht werden, so beschrieben in P. C. Levendag, L. L. Visch, N. Doiver, The Journal of Prosthetic Dentistry, 63 (6), (1990), S. 665.

Vor diesem Hintergrund war die Erfindung eines mit Kathetern ausgerüsteten Flabs gemäß US-PS 4 963 128 ein Fortschritt, der aber den Anforderungen der Praxis noch nicht im vollen Umfang genügt. Die Krümmungsradien der äußeren Katheter sind eng und gefährden die Beweglichkeit der Quellen. Die Ösen zum Fixieren des sogenannten Flabs sind leicht einreißbar und können häufig nicht in vollem Umfang zur Fixierung genutzt werden, da sie auch in nicht-nähbaren Bereichen lokalisiert sein können. Darüber hinaus gestattet das extrem weiche Material des Flabs auch über die vier Ösen keine grundsätzlich sichere Fixierung. Weiterhin muß aufgrund der unterschiedlichen geometrischen Ausdehnung der zu behandelnden Flächen eine kostenaufwendige Lagerhaltung verschieden großer, derartig mit Kathetern ausgerüsteter Flabs vorgesehen werden.

Die Flabs des Standes der Technik sind ausschließlich für flach ausgedehnte oder nur mäßig gekrümmte Behandlungsstellen verwendbar. Für engere Körperhöhlen oder stark gekrümmte Körper und Gewebeflächen sind Flabs dieser Bauart grundsätzlich nicht geeignet.

Aufgabe der vorliegenden Erfindung war es, sowohl flache als auch für die üblichen Körperhöhlen sowie für stark gekrümmte Behandlungsflächen geeignete Kunststoff-Katheter-Systeme für die Strahlentherapie nach der Afterloading-Methode zu entwickeln, die o. g. Nachteile nicht aufweisen und ohne nennenswerten Aufwand an die Applikationsbedingungen durch einfaches Zuschneiden angepaßt werden können. Die erfindungsgemäßen Kunststoff-Katheter-Systeme zeichnen sich aus, daß sie bei flacher Ausführung oberhalb der Katheter über die gesamte Fläche und auf der von der Bestrahlung abgewandten Seite mit einem Kunststoffgewebe und im Falle zylindrischer Formen im unteren Teil, bei anderen Formen stets auf der von der Bestrahlung abgewandten Seite, mit einem Kunststoffgewebe ausgerüstet sind und die Kunststoff-Katheter-Systeme gemäß Bedarf und Anforderungen beliebig zugeschnitten werden können. Dabei war es erforderlich, daß der die Katheter umgebende Kunststoff weich und flexibel ist, ein flüssigkeitsdichter Kontakt zwischen Kathetern und Kunststoff-Matrix sichergestellt ist, das Kunststoff-Material Dichten von 0,9 bis 1,2 g/cm³ besitzt und gemessen an der Applikationsdauer und der Schwere der Indikation nicht gesundheitsschädigend ist sowie nach den üblichen Verfahren einer Sterilisation unterzogen werden kann. Weiterhin kennzeichnend für die vorliegende Erfindung ist die Ausrüstung dieser Kunststoff-Katheter-Systeme mit einem unter der Oberfläche eingearbeiteten, nicht fasernden, körperfreundlichen Kunststoff-Gewebe über die gesamte Fläche oder einem koaxialen Kunststoffaser-Gewebe am unteren Teil der zylindrischen Körper, in allen Fällen auf der von der Bestrahlungsfläche abgewandten Seite oder abseits von dem Bestrahlungsbereich (s. Abb.).

Des weiteren kennzeichnet die vorliegende Erfindung, daß es möglich ist auf der der Bestrahlungsfläche abgewandten Seite in das Kunststoffmaterial Stoffe mit hoher Ordnungszahl (z. B. Blei) so einzuarbeiten, daß die Flexibilität des Materials erhalten bleibt und eine abschirmende Wirkung gegenüber der verwendeten Strahlenquelle eintritt, die eine Reduktion der Strahlenexposition in diesem Bereich um bis zu 80 % bewirkt. Als Folge hiervon kann die Strahlenexposition des Patienten im wesentlichen auf den eigentlichen Herd beschränkt und im Falle einer allfälligen Bergung des Patienten die Strahlenexposition des Personals entscheidend reduziert werden.

Als Kunststoff-Materialien zur Einbettung der Afterloading-Katheter sind körperfreundliche, weiche Kunststoffe auf Basis von Polyurethanen, Polyolefinen, Polycarbonaten, Polyvinylchlorid, Polysulfonen, Polyethern, Polyestern, Polyamiden und analogen Polymeren und Siliconen mit und ohne Weichmachern einsetzbar, deren Dichte im Bereich von 0,8 bis 1,5 g/cm³ liegt, vorzugsweise von 0,9 bis 1,2 g/cm³, eine reproduzierbare, möglichst geringe γ-Strahlenabsorption besitzen und ohne Bildung schädlicher Zersetzungsprodukte nach gängigen Verfahren sterilisierbar sind. Grundsätzlich sind auch Gel-artige Polymersysteme geeignet. Die Katheter bestehen vorzugsweise aus Polyamid, andere Materialien sind aber ebenfalls denkbar.

Das in den Kunststoff eingearbeitete Gewebe muß aus nicht-fasernden, gewebeverträglichen Polymerfasern bestehen, die sich gut schneiden lassen und mit der umgebenden Kunststoff-Matrix einen festen, hohlraum- und rißfreien Verbund geben. Verwendbar sind Gewebe aus den o. g. Polymeren und anderen; Glasfaser-Gewebe sind dagegen wegen ihrer Neigung zur Bildung feiner Bruchstücke, die zu unerwünschten Nebenreaktionen führen und nur schwer aus dem Gewebe entfernt werden können, nicht geeignet. Die Kunststoffgewebe sind mit dem Körpergewebe vernähbar.

Die erfindungsgemäßen Kunststoff-Katheter-Systeme lassen sich in den Grundformen direkt einsetzen oder auch beliebig zuschneiden, wobei auf einen geraden Schnitt bei den Kathetern zu achten ist. Bei Verwendung von flexiblen Hülsen müssen, als Platzhalter für die Katheter im Flab, die Katheter in diese eingeführt werden und sind dann bündig im Führungsrohr festzukleben. Die offenen Katheterenden sind mit einem Pfropfen aus gleichem Material zu verschließen. Dies erfolgt entweder durch Erhitzen des Pfropfens bis zum Einweichungspunkt gemäß Vorschrift des Katheterherstellers, oder sie sind mit einer erhitzten Flachzange so zu schließen, daß ein rechtwinkliger Verschluß ohne zusätzliche Einengung erfolgt. Auch ein Verschließen mit einem gewebeverträglichen Binder wie z. B. mit Scotch-Weld DP 490 (3 M) oder Vitralit 6127 (Panacol Elosol) ist möglich.

Ein weiter wesentlicher Vorteil der erfindungsgemäßen Kunststoff-Katheter-Systeme ist, daß sie bei jeder geänderten geometrischen Form an jeder Stelle fixierbar sind und durch das eingesetzte Gewebe durchgängige Rißbildungen in den Durchstichen ausgeschlossen sind und somit ein Verrutschen vermieden wird.

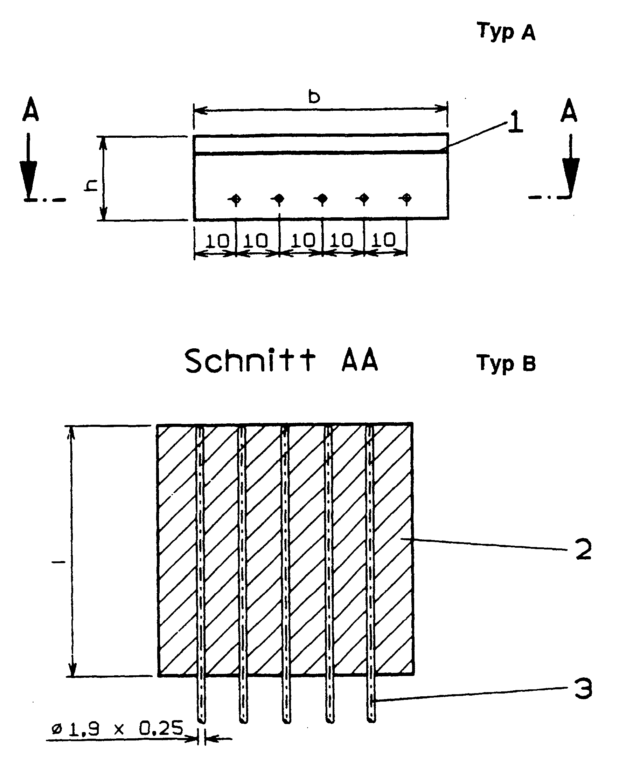

Die erfindungsgemäßen Kunststoff-Katheter-Systeme sind in den Abbildungen 1 - 3 in ihren grundsätzlichen Formen A bis I und Standard-Abmessungen dargestellt.

Für die flachen Kunststoff-Katheter-Systeme treffen Abmessungen von 100 x 100 mm und 300 x 300 mm sowie Dicken von 3, 5, 10 und 20 mm (Typ A und B in Abb. 1) auf das größte Anwenderinteresse. Die eingebetteten Katheter können im geschlossenen Zustand unmittelbar an der Kunststoffkante enden oder durch die Kunststoffmatrix durchlaufen und außerhalb offen enden.

Letzteres kann vor allem zutreffen, wenn die erfindungsgemäßen Kunststoff-Katheter-Systeme in Serie unter Verwendung von Endlos-Kathetern hergestellt werden. Die offene Seite ist vor Gebrauch anwendungsgerecht einzukürzen und zu verschließen. Die für die Bestrahlung vorgesehene Seite befindet sich unterhalb der Katheteranordnung.

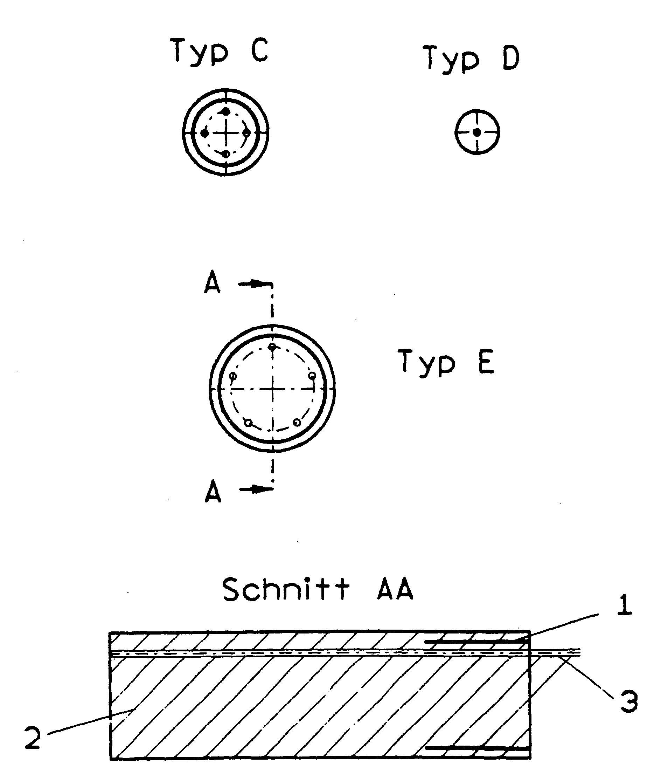

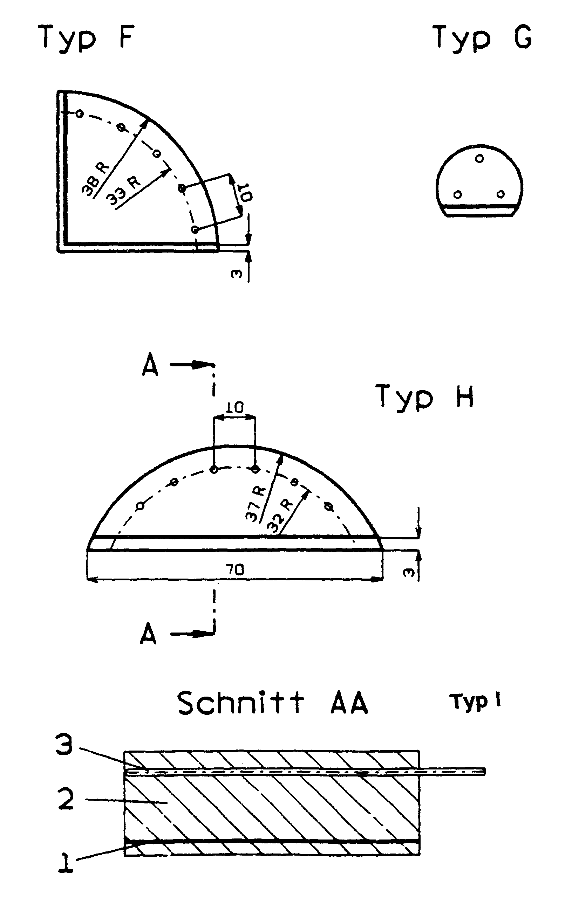

Die Abbildung 2 zeigt zylindrische Kunststoff-Katheter-Systeme unterschiedlicher Abmessungen, die strahlentherapeutischen Applikationen entsprechen und gemäß Bedarf als Standardtypen hergestellt werden. Grundsätzlich gehört auch der Typ I (Abb. 3) dazu, hier nur in der Draufsicht dargestellt. Alle diese zylindrischen Typen C - E und Typ I sind einseitig an der Katheter-Eintrittsseite mit einem koaxialen, zylindrischen Gewebeeinsatz ausgerüstet. Auch bei diesen Kunststoff-Katheter-Typen endet der Katheter einseitig geschlossen an der Kunststoffmatrix-Stirnfläche oder durchläuft sie und endet offen.

Die Typen F - H in Abbildung 3 sind beispielhafte Sonderformen, die als Basiskörper für spezielle geformte Körperhöhlen geeignet sind.

Ausgehend von den Basistypen A bis I sind auch beliebig andere Formen möglich, etwa nach Vorlage eines Modellkörpers und auch mit einem nichtgeradlinigen, jedoch exakt festgelegten Verlauf der Katheter; gemäß vorliegender Erfindung sind auch solche Formen durch Zuschnitt exakt anpaßbar und grundsätzlich mit einer Polymergewebe-Einlage ausgerüstet.

### . Applikationsbeispiele:

1. 25-jähriger junger Mann mit einem extraossären, kleinzelligen, malignen Tumor, am ehesten PNET am medianen, linken Oberschenkel. Zustand nach 6 Kursen Chemotherapie, Zustand nach präoperativer Radiatio des linken Oberschenkels bis 54 Gy.
   **Jetzt:** Nach Tumorverkleinerung durch Vortherapie en bloc-Resektion des Tumors mit anhängender Knochenlamelle und intraoperativer high-doserate-afterloading-Radiatio mittels Iridium-192 im Bereich des Tumorbettes.
   Es wurde eine einmalige Dosis von 10 Gy auf 0,5 cm Gewebetiefe dosiert, von cranial beschickt und insgesamt eine Länge von 18 cm hatte.
   Der Patient hat, soweit uns bekannt, die Radiatio ohne Komplikationen gut vertragen. Es traten keine Infektionen und keine Wundheilungsstörungen auf.
2. 34-jähriger junger Mann mit einem Mucoepidermoidcarcinom im Bereich des Zungengrundes, primär inoperabel. Zustand nach perkutaner Radiatio bis 70 Gy. Zustand nach interstitieller Brachytherapie mit einer Gesamtherddosis von 10 Gy.
   **Jetzt:** Tumorprogression nach abgeschlossener Radiatio. Zur lokalen Dosisaufsättigung wurde eine 2-malige Radiatio des Zungengrundes und der Hypopharynxregion mittels einer Flab-Applikation durchgeführt. Hierbei wurde 2 x 5 Gy über eine Distanz von 5 cm, bezogen auf 0,5 cm Gewebetiefe, appliziert.
   Der Patient hat die Bestrahlung ohne wesentliche Nebenwirkungen gut vertragen.

### Erläuterungen zu den Abbildungen:

Die hier vorgestellten Kunststoff-Katheter-Systeme sind nur beispielhaft in ihren Abmessungen aufgeführt und keineswegs auf diese beschränkt.

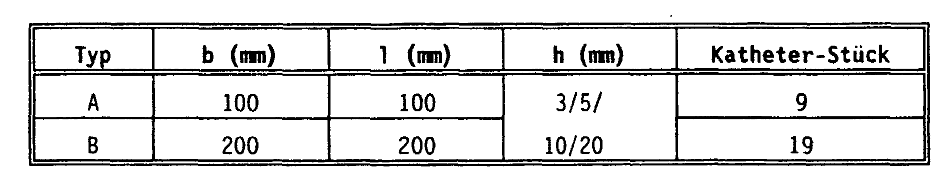

| **Teil Nr.** | **Benennung** |
|---|---|
| 1 | Gewebe z. B. 100 x 0,4 x 100 |
| 2 | Weich Polymer z. B. 100 x 20 x 100 |
| 3 | Katheter z. B. 1,9 x 0,25 x 400 und länger |

| **Typ** | **Teil Nr.** | **Benennung** |
|---|---|---|
| C | 1 | Gewebe z. B. 50 x 0,4 x 25 |
| | 2 | Weich Polymer z. B. 0̸ 20 x 100 |
| | 3 | Katheter z. B. 1,9 x 0,25 x 100 und länger |
| D | 1 | Gewebe z. B. 30 x 0,4 x 25 |
| | 2 | Weich Polymer z. B. 0̸ 10 x 200 |
| | 3 | Katheter z. B. 1,9 x 0,25 x 400 und länger |
| E | 1 | Gewebe z. B. 81 x 0,4 x 25 |
| | 2 | Weich Polymer z. B. 0̸ 30 x 200 |
| | 3 | Katheter z. B. 1,9 x 0,25 x 400 und länger |
| F - I | 1 | Gewebe z. B. 65 x 0,4 x 65 |
| | 2 | Weich Polymer z. B. 70 x 20 x 90 |
| | 3 | Katheter z. B. 1,9 x 0,25 x 400 und länger |

## Patentansprüche

1. Flexible Kunststoff- Körper (2) mit Einzelkathetern oder in der Regel äquidistant eingebetteten Kathetern (3) oder Hülsen für die Strahlentherapie, vorzugsweise Brachytherapie, nach der Afterloading-Methode,
**dadurch gekennzeichnet,**
**dass** die Kunststoff- Katheter- Systeme von flacher Gestalt sind, oberhalb der Katheter mit einem unter der Oberfläche des Kunststoffs eingearbeiteten, nicht fasernden körperfreundlichen Kunststofffaser-Gewebe (1), welches die gesamte ebene Fläche einnimmt, ausgerüstet sind, wobei das Kunststofffaser-Gewebe (1) mit dem Körpergewebe vernähbar ist.

2. Kunststoff-Katheter-Systeme gemäß Anspruch 1,
**dadurch gekennzeichnet,**
**dass** deren Kunststoffmatrix weich und flexibel ist und eine Dichte von 0,8 bis 1,5 g/cm³, vorzugsweise 0,9 und 1,2 g/cm³, besitzt.

3. Kunststoff-Katheter-Systeme gemäß den Ansprüchen 1 bis 2,
**dadurch gekennzeichnet,**
**dass** die Kunststoffe aus Polyurethanen, Polyolefinen, Polycarbonaten, Polyvinylchlorid, Polysulfonen, Polyethern, Polyestern, Polyamiden und analogen Polymeren sowie Siliconen mit und ohne Weichmachern bestehen.

4. Kunststoff-Katheter-Systeme gemäß Anspruch 1,
**dadurch gekennzeichnet**,
Kunststofffaser-Gewebe dass deren (1) sich über die gesamte ebene Fläche, bei der Fixierung im oder am Körper von der Bestrahlungsseite abgewandt, befinden, und aus nicht-fasernden, schneidfähigen, gewebeverträglichen Polymerfasern, vorzugsweise Fasermaterial der Polymere gemäß Anspruch 3, bestehen.

5. Kunststoff-Katheter-Systeme gemäß den Ansprüchen 1 bis 4,
**dadurch gekennzeichnet,**
**dass** bei der Fixierung im oder am Körper auf der Bestrahlungsfläche abgewandten Seite Material zur Abschirmung der Strahlung verwendet wird.

6. Flexible Kunststoff- Körper (2) mit Einzelkathetern oder in der Regel äquidistant eingebetteten Kathetern (3) oder Hülsen für die Strahlentherapie, vorzugsweise Brachytherapie, nach der Afterloading-Methode,
**dadurch gekennzeichnet,**
**dass** die Kunststoff- Katheter- Systeme von zylindrischer Gestalt sind, mit einem unter der Oberfläche des Kunststoffs eingearbeiteten, nicht fasernden körperfreundlichen Kunststoffaser-Gewebe (1) ausgerüstet sind, wobei die Gewebeeinlagen koaxial auf den unteren Teil, wo die Katheter oder der einzelne Katheter in die einbettende Kunststoff-Matrix eintreten, beschränkt sind und das Kunststofffaser-Gewebe mit dem Körpergewebe vernähbar ist.

7. Kunststoff-Katheter-Systeme gemäß Anspruch 6,
**dadurch gekennzeichnet,**
**dass** deren Kunststoffmatrix weich und flexibel ist und eine Dichte von 0,8 bis 1,5 g/cm³, vorzugsweise 0,9 und 1,2 g/cm³, besitzt.

8. Kunststoff-Katheter-Systeme gemäß den Ansprüchen 6 bis 7,
**dadurch gekennzeichnet,**
**dass** die Kunststoffe aus Polyurethanen, Polyolefinen, Polycarbonaten, Polyvinylchlorid, Polysulfonen, Polyethern, Polyestern, Polyamiden und analogen Polymeren sowie Siliconen mit und ohne Weichmachern bestehen.

9. Kunststoff-Katheter-Systeme gemäß Anspruch 6,
**dadurch gekennzeichnet**,
Kunststofffaser-Gewebe dass deren (1) sich über die gesamte ebene Fläche bei der Fixierung im oder am Körper von der Bestrahlungsseite abgewandt, befinden, und aus nicht-fasernden, schneidfähigen, gewebeverträglichen Polymerfasern, vorzugsweise Fasermaterial der Polymere gemäß Anspruch 8, bestehen.

10. Kunststoff-Katheter-Systeme gemäß den Ansprüchen 6 bis 9,
**dadurch gekennzeichnet,**
**dass** bei der Fixierung im oder am Körper auf der Bestrahlungsfläche abgewandten Seite Material zur Abschirmung der Strahlung verwendet wird.

## Claims

1. A flexible plastics body (2) with individual catheters or with catheters (3) or sheaths which are, as a rule, embedded at equal distances, for radiotherapy, preferably brachytherapy, by the afterloading method, **characterized in that** the plastics catheter system has a flat shape, and is equipped above the catheters with a non-fluffing, biocompatible synthetic fibre fabric (1) which is incorporated under the surface of the plastics material and which occupies the entire flat area, the synthetic fibre fabric (1) being able to be sutured to body tissue.

2. A plastics catheter system according to claim 1, **characterized in that** the plastics matrix thereof is soft and flexible and has a density of from 0.8 to 1.5 g/cm³, preferably 0.9 to 1.2 g/cm³.

3. A plastics catheter system according to claim 1 or 2, **characterized in that** the plastics materials consist of polyurethanes, polyolefins, polycarbonates, polyvinyl chloride, polysulphones, polyethers, polyesters, polyamides and analogous polymers, and silicones, with and without plasticizers.

4. A plastics catheter system according to claim 1, **characterized in that** its synthetic fibre fabric (1) is located over the entire flat area during fixation in or on the body facing away from the irradiation side and consists of non-fluffing, cuttable, tissue-compatible polymer fibres, preferably fibre material of the polymers according to claim 3.

5. A plastics catheter system according to any of claims 1 to 4, **characterized in that** during fixation in or on the body a material for shielding radiation is used on the side facing away from the irradiation area.

6. A flexible plastics body (2) with individual catheters or with catheters (3) or sheaths which are, as a rule, embedded at equal distances, for radiotherapy, preferably brachytherapy, by the afterloading method, **characterized in that** the plastics catheter system has a cylindrical shape, and is equipped above the catheters with a non-fluffing, biocompatible synthetic fibre fabric (1) which is incorporated under the surface of the plastics material, the fabric inserts being confined coaxially to the lower part where the catheters or the individual catheter enter into the embedding plastics matrix, the synthetic fibre fabric being able to be sutured to body tissue.

7. A plastics catheter system according to claim 6, **characterized in that** the plastics matrix thereof is soft and flexible and has a density of from 0.8 to 1.5 g/cm³, preferably 0.9 to 1.2 g/cm³.

8. A plastics catheter system according to claim 6 or 7, **characterized in that** the plastics materials consist of polyurethanes, polyolefins, polycarbonates, polyvinyl chloride, polysulphones, polyethers, polyesters, polyamides and analogous polymers, and silicones, with and without plasticizers.

9. A plastics catheter system according to claim 6, **characterized in that** its synthetic fibre fabric (1) is located over the entire flat area during fixation in or on the body facing away from the irradiation side and consists of non-fluffing, cuttable, tissue-compatible polymer fibres, preferably fibre material of the polymers according to claim 8.

10. A plastics catheter system according to any of claims 6 to 9, **characterized in that** during fixation in or on the body a material for shielding radiation is used on the side facing away from the irradiation area.

## Revendications

1. Pièce en matière plastique souple (2) avec des cathéters séparés ou des cathéters (3) intégrés en général de manière équidistante ou des manchons pour la radiothérapie et notamment la brachythérapie selon le procédé de recharge,
**caractérisée en ce que**
les systèmes matière plastique-cathéter sont de forme plate et au-dessus des cathéters ils ont un tissu (1) de fibres de matière plastique, ne fibrant pas, accepté par le corps humain, ce tissu étant intégré sous la surface de la matière plastique et il occupe toute la surface plane, le tissu de fibres de matière plastique (1) pouvant être cousu au tissu corporel.

2. Système matière plastique-cathéter selon la revendication 1,
**caractérisé en ce que**
sa matrice de matière plastique est souple et molle et a une densité de 0,8 à 1,5 g/cm³, de préférence 0,9 et 1,2 g/cm³.

3. Système matière plastique-cathéter selon les revendications 1 et 2,
**caractérisé en ce que**
les matières plastiques sont choisies parmi les polyuréthanes, polyoléfines, polycarbonates, polychlorure de vinyle, polysulfones, polyéthers, polyesters, polyamides et polymères analogues ainsi que des silicones avec ou sans plastifiant.

4. Système matière plastique-cathéter selon les revendications 1 et 2,
**caractérisé en ce que**
son tissu de fibres en matière plastique (1) s'étend sur toute la surface plane et pour la fixation dans ou sur le corps humain, on l'installe du côté opposé au côté du rayonnement, et ce tissu se compose de fibres de polymère acceptées par le tissu humain, qui ne se défibrent pas et peuvent être coupées, de préférence une matière fibreuse de polymère selon la revendication 3.

5. Système matière plastique-cathéter selon les revendications 1 à 4,
**caractérisé en ce que**
pour la fixation dans ou sur le corps, le côté opposé à la surface de rayonnement comporte une matière de protection contre le rayonnement.

6. Elément souple en matière plastique (2) avec des cathéters séparés ou intégrés en général de manière équidistante (3) ou des manchons pour la radiothérapie, de préférence la brachythérapie selon le procédé de recharge,
**caractérisé en ce que**
les systèmes matière plastique-cathéter sont de forme cylindrique avec un tissu de fibres de matière plastique (1) ne libérant pas les fibres, accepté par le corps humain, et qui sont intégrées sous la surface de la matière plastique, les inserts de tissu étant limités à la partie inférieure là où les cathéters ou les différents cathéters pénètrent dans la matrice de matière plastique, et le tissu de fibres de matière plastique peut être cousu au tissu corporel.

7. Système matière plastique/cathéter selon la revendication 6,
**caractérisé en ce que**
sa matrice de matière plastique est molle et souple et a une densité de 0,8 à 1,5 g/cm³, de préférence 0,9 et 1,2 g/cm³.

8. Système matière plastique/cathéter selon les revendication 6 et 7,
**caractérisé en ce que**
les matières plastiques sont choisies parmi les polyuréthanes, polyoléfines, polycarbonates, polychlorure de vinyle, polysulfones, polyéthers, polyesters, polyamides et polymères analogues ainsi que des silicones avec ou sans plastifiant.

9. Système matière plastique/cathéter selon les revendication 6,
**caractérisé en ce que**
le tissu de fibres de matière plastique (1) s'étend sur toute la surface plane du côté opposé au côté du rayonnement pour la fixation dans ou sur le corps et de fibres de polymère acceptées par le tissu, ne libérant pas de fibres et pouvant se couper, de préférence une matière fibreuse de polymère selon la revendication 8.

10. Système matière plastique/cathéter selon les revendication 6 à 9,
**caractérisé en ce que**
pour la fixation dans ou sur le corps, le côté de la matière à l'opposé de la surface de rayonnement comporte un écran pour le rayonnement.
